# EUROPÄISCHE PATENTSCHRIFT

(11) **EP 0 667 348 B1**
(45) Veröffentlichungstag und Bekanntmachung des Hinweises auf die Patenterteilung: **10.05.2000**
(21) Anmeldenummer: 95101387.9
(22) Anmeldetag: 02.02.1995
(51) Int. Cl.: C07D 471/14

(54) **Verfahren zur Herstellung von Nevirapine**
Process for the preparation of nevirapine
procédé pour la préparation de névirapine

(30) Priorität: 03.02.1994 DE 4403311
(43) Veröffentlichungstag der Anmeldung: 16.08.1995
(73) Patentinhaber: Boehringer Ingelheim Pharma KG, 55216 Ingelheim am Rhein (DE); BOEHRINGER INGELHEIM INTERNATIONAL GmbH, 55218 Ingelheim am Rhein (DE)
(72) Erfinder: Schneider, Heinrich, Dr. Dipl.-Chem., D-55218 Ingelheim am Rhein (DE); Christmann, Albrecht, Dr. Dipl.-Chem., D-55218 Ingelheim am Rhein (DE)
(74) Vertreter: Laudien, Dieter, Dr.

(56) Entgegenhaltungen:
- EP-A- 0 393 529
- EP-A- 0 410 148
- EP-A- 0 429 987
- EP-A- 0 482 481
- JOURNAL OF HETEROCYCLIC CHEMISTRY, Bd. 30, 1993 Seiten 771-779, M.H. NORMAN ET AL. 'Structural Elucidation of an Oxazolo[5,4-b]pyridine: an Alternative Cyclization Product Related to Nevirapine'
- JOURNAL OF MEDICINAL CHEMISTRY, Bd. 34, 1991 Seiten 2231-2241, K.D. HARGRAVE ET AL. 'Novel Non-Nocleoside Inhibitors of HIV-1 Reverse Transcriptase. 1. Tricyclic Pyridobenzo- and Dipyridodiazepinones'

## Beschreibung

Die vorliegende Erfindung betrifft ein verbessertes Verfahren zur Herstellung des Virustatikums Nevirapine (11-Cyclopropyl-5,11-dihydro-4-methyl-6-H-dipyrido[3,2-b:2',3'-e][1,4-diazepin]-6-on)

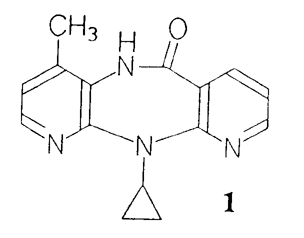

Nevirapine verkörpert einen nicht-nukleosidischen Inhibitor der HIV-1-Reverse-Transscriptase.

Aus dem Stand der Technik ist ein Verfahren zur Herstellung von Nevirapine bekannt, bei dem 2-Chlor-3-pyridincarbonylchlorid (2) mit 3-Amino-2-chlor-4-methylpyridin (3) umgesetzt wird

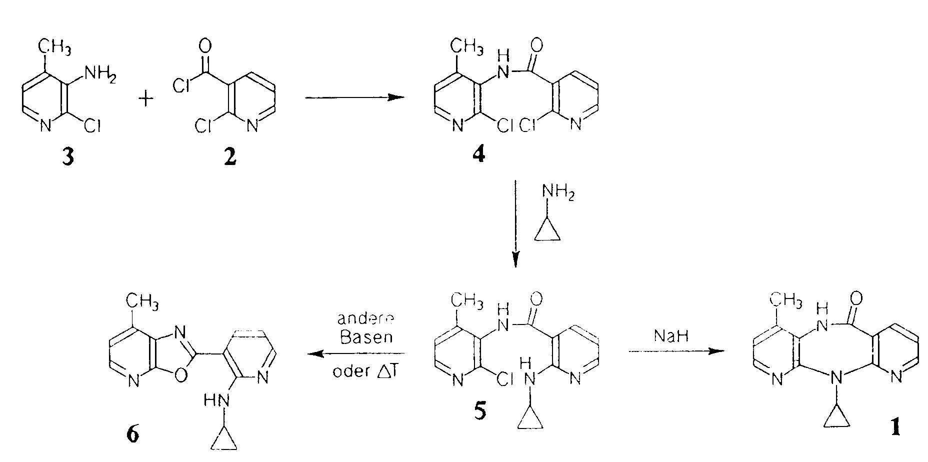

[K. D. Hargrave et al., J. Med. Chem. 34, 2231 (1991) und M.H. Norman, D.J.Minick und G.E. Martin, J. Heterocyclic Chem. 30, 771 (1993)], wobei das Acylierungsagenz durch Umsetzung von 2-Chlor-3-pyridin-carbonsäure mit Thionylchlorid zugänglich ist. Aus dieser Umsetzung resultiert das 2-Chlor-N-(2-chlor-4-methyl-3-pyridinyl)-3-pyridincarboxamid (4), welches mit Cyclopropylamin zum N-(2-Chlor-4-methyl-3-pyridyl)-2-(cyclopropylamino)-3-pyridincarboxamid (5) umgesetzt wird. Auf der letzten Reaktionsstufe erfolgt dann mittels Natriumhydrid die Cyclisierung zum Nevirapine (1). Eine entsprechende Vorgehensweise wird auch durch die Europäische Patentanmeldung EP 482 481 offenbart und findet ebenfalls Anwendung bei der Synthese strukturell mit Nevirapine verwandter Verbindungen (z.B. EP 393 529).

Der Nachteil dieses aus dem Stand der Technik bekannten Verfahrens besteht u.a. darin, daß bei der Herstellung des N-(2-Chlor-4-methyl-3-pyridyl)-2-(cyclopropylamino)-3-pyridin-carboxamids (5) zum einen ein großer Überschuß (ca. 400 mol-%) an Cyclopropylamin eingesetzt werden muß, andernfalls entstehen bei dieser Umsetzung Nebenprodukte - wie zum Beispiel die Verbindung der Struktur 6 -. welche in der anschließenden - letzten - Reaktionsstufe unerwünscht sind.

Überraschenderweise wurde nun gefunden, daß sich einerseits der große Überschuß des - relativ teuren - Cyclopropylamins vermindern läßt, wenn die Umsetzung in Gegenwart von Calciumoxid (CaO) durchgeführt wird. Durch Zugabe von 0,5 bis 2.5 Mol Calciumoxid läßt sich der Einsatz von Cyclopropylamin bei der Umsetzung von 2-Chlor-N-(2-chlor-4-methyl-3-pyridyl)-3-pyridincarboxamid (4) zum N-(2-Chlor-4-methyl-3-pyridyl)-2-(cyclopropylamino)-3-pyridincarboxamid (5) deutlich auf ein Verhältnis von 2 bis 2,5 mol Cyclopropylamin, bezogen auf ein Mol des Edukts 4, reduzieren. Daneben entstehen bei der Umsetzung weniger Verunreinigungen bzw. Nebenprodukte. Auf der anderen Seite werden im nachfolgenden Cyclisierungsschritt zum Nevirapine 10 % weniger an der Base Natriumhydrid benötigt. Ein weiterer Vorteil des erfindungsgemäßen Verfahrens besteht darin, daß das Lösungsmittel auf destillativem Wege zurückgewonnen und für einen neuen Reaktionslauf eingesetzt werden kann. Dabei hat sich gezeigt, daß beim Einsatz von Diglyme als Reaktionsmedium das o.a. Destillat noch ca. 90 Mol-% Cyclopropylamin enthält, was eine Ersparnis an Cyclopropylamin mit sich bringt, wenn das Destillat beim nächsten Ansatz als Lösungsmittel eingesetzt wird. Daneben läßt sich die Reaktion ohne Verschlechterung der Produktqualität auch im großtechnischen Maßstab durchführen.

Hierbei ergibt sich ein weiterer technischer Vorteil: Für den Fall einer nicht mehr kontrollierbaren Reaktion - z.B. bei einem Kühlmittelausfall - kann bei Verwendung von 400 Mol-% Cyclopropylamin der Druck bis auf 28-30 bar ansteigen, bei Verwendung von 250 Mol-% jedoch nur auf 8-9 bar, so daß nur noch Apparate mit weit niedrigerer Druckstufe benötigt werden.

Ein zusätzlicher Vorteil läßt sich erzielen, wenn nur zunächst 100-150 Mol-% Cyclopropylamin für ca. 30 Minuten zur Reaktion gebracht werden und erst dann das restliche Cyclopropylamin zugepumpt wird. In diesem Fall ist die Verwendung von Rührwerksapparaten nach DIN 28136 möglich, die standardmäßig bis 6 bar zugelassen sind (Beispiel 3).

Die Herstellung des N-(2-chlor-4-methyl-3-pyridyl)-2-(cyclopropylamino)-3-pyridincarboxamids (5) und die nachfolgende Cyclisierung zum 11-Cyclopropyl-5,11-dihydro-4-methyl-6H-dipyrido-[3,2-b : 2',3'-e][1,4]diazepin-6-on (1) kann im großtechnischen Maßstab beispielsweise - wie folgt - durchgeführt werden:

Ein Gemisch von 2-Chlor-N-(2-chlor-4-methyl-3-pyridyl)-3-pyridincarboxamid (4), Calciumoxid und Cyclopropylamin (100 : 100 : 250 mol-%) in Diglyme (Diethylenglykol-dimethylether), wird in einem Autoklaven aus VA-Stahl über einen Zeitraum von 5 bis 8 Stunden, bevorzugt 6 bis 7 Stunden, auf eine Temperatur in einem Intervall von 130 bis 150°C, bevorzugt 135 bis 145°C erhitzt.

Danach wird das Reaktionsgemisch auf eine Temperatur unter 40 °C, bevorzugt auf eine Temperatur im Bereich von 20 bis 30°C abgekühlt und filtriert. Der Filterkuchen wird mit Diglyme gewaschen. Die vereinigten Filtrate werden eingeengt und anschließend wieder mit Diglyme verdünnt. Die so erhaltene Lösung wird zu einer auf eine Temperatur im Bereich von 120 bis 150°C - vorzugsweise 130°C - erhitzten Suspension oder Lösung einer Base - vorzugsweise einer 60-proz. Suspension von Natriuimhydrid in Diglyme - zudosiert und über einen Zeitraum von 0,5 bis 1,5 Stunden - bevorzugt 0,5 bis 1,0 Stunden - bei einer Temperatur in einein Intervall von 120 bis 150°C, bevorzugt 130 bis 140°C, gerührt. Danach werden das Reaktionsmedium und die flüchtigen Bestandteile der Reaktionsmischung weitestgehend abdestilliert. Der verbliebene Rückstand wird bei einer Temperatur im Bereich von 20 bis 90°C, vorzugsweise 50 bis 80°C, vorsichtig hydrolysiert. Nach dem Abkühlen auf eine Temperatur von ca. 25°C wird das Hydrolysat mit einem inerten Lösungsmittel, beispielsweise einem Kohlenwasserstoff, vorzugsweise Cyclohexan und einer organischen Säure, vorzugsweise Eisessig, versetzt und über einen Zeitraum von 0,5 bis 1,5 Stunden, vorzugsweise über einen Zeitraum von einer Stunde, bei einer Temperatur in einem Intervall von 5 bis 50°C, vorzugsweise in einem Intervall von 10 bis 25°C, gerührt. Die resultierende Suspension wird anschließend geschleudert und das Schleudergut mit einem inerten Lösungsmittel - wie beispielsweise einem Dialkylether oder einem Kohlenwasserstoff- bevorzugt Methyl-tert.-butylether - und anschließend mit Wasser sowie abschließend mit einem Alkanol, bevorzugt Ethanol, gewaschen.

Die zuvor beschriebenen Vorteile des erfindungsgemäßen Verfahrens werden durch die in den Beispielen beschriebenen Verfahrensweisen gelöst. Verschiedenartige, andere Ausgestaltungen des Verfahrens werden für den Fachmann aus der vorliegenden Beschreibung ersichtlich. Es wird jedoch ausdrücklich darauf hingewiesen, daß die Beispiele und die diesen zugeordnete Beschreibung lediglich zum Zweck der Erläuterung und Beschreibung vorgesehen und nicht als Einschränkung der Erfindung anzusehen sind.

### Beispiel 1

117.5 kg 2-Chlor-N-(2-chlor-4-methyl-3-pyridyl)-3-pyridincarboxamid (4), 23.3 kg Calciumoxid und 59.4 kg Cyclopropylamin (Molverhältnis: 1:1:2,5) werden in 235 l Diglyme (Diethylenglykol-dimethylether) in einem 500 l-VA-Autoklaren über einen Zeitraum von 6 bis 8 h auf 135 bis 145°C erhitzt. Anschließend wird das Reaktionsgemisch auf eine Temperatur von 20 bis 30°C abgekühlt und filtriert. Der Filterkuchen wird mit 58.8 l Diglyme gewaschen. Die Filtrate werden vereinigt und es werden zunächst 200 l Lösungsmittel abdestilliert. Der Rückstand wird daraufhin mit weiteren 117.5 l Diglyme verdünnt. Die so erhaltene verdünnte Lösung wird innerhalb eines Zeitraumes von 20 - 40 min. zu einer 130°C warmen Suspension von 45.0 kg 60-proz. Natriumhydrid in 352.5 l Diglyme zudosiert. Vorratsgefäß und Leitungen werden mit weiteren 55.8 l Diglyme gespült und das Gemisch wird unter Rühren über einen weiteren Zeitraum von 30 bis 60 min. auf einer Temperatur im Bereich von 130-140°C gehalten. Danach wird das Diglyme weitgehend abdestilliert; anschließend wird der verbliebene Rückstand vorsichtig mit 470 l Wasser versetzt. Nach dem Abkühlen des Reaktionsgemisches auf eine Temperatur von ca. 25°C werden 235.0 l Cyclohexan und 57.1 l Eisessig zugefügt. Es wird nach ca. 1 h bei einer Temperatur in einem Intervall von 10 - 25°C nachgerührt. Die resultierende Suspension wird geschleudert und das Schleudergut anschließend mit 235.0 l Methyl-tert.-butylether und daraufhin mit 353.5 l Wasser und abschließend mit 235 l Ethanol gewaschen. Man isoliert auf diese Weise nach dem Trocknen 92.5 kg (83.5 % d. Th.) des 11-Cyclopropyl-5,11-dihydro-4-methyl-6H-dipyrido[3,2-b:2',3'-e][1,4]diazepin-6-ons (Nevirapine).

### Beispiel 2

117.5 kg 2-Chlor-N-(2-chlor-4-methyl-3-pyridyl)-3-pyridincarboxamid (4), 46.7 kg Calciumoxid und 47.5 kg Cyclopropylamin (Molverhältnis: 1:2:2) werden in 235 l Diglyme (Diethylenglykol-dimethylether) in einem 500 l-VA-Autoklaren über einen Zeitraum von 6 bis 8 h auf 135 bis 145°C erhitzt. Anschließend wird das Reaktionsgemisch auf eine Temperatur von 20 bis 30°C abgekühlt und filtriert. Der Filterkuchen wird mit 58.8 l Diglyme gewaschen. Die Filtrate werden vereinigt und es werden zunächst ca. 188 l Lösungsmittel abdestilliert. Der Rückstand wird daraufhin mit weiteren 117.5 l Diglyme verdünnt. Die so erhaltene verdünnte Lösung wird innerhalb eines Zeitraumes von 20 - 40 min. zu einer 130°C warmen Suspension von 45.0 kg 60-proz. Natriumhydrid in 352.5 l Diglyme zudosiert. Vorratsgefäß und Leitungen werden mit weiteren 55.8 l Diglyme gespült und das Gemisch wird unter Rühren über einen weiteren Zeitraum von 30 bis 60 min. auf einer Temperatur im Bereich von 130-140°C gehalten. Danach wird das Diglyme weitgehend abdestilliert; anschließend wird der verbliebene Rückstand vorsichtig mit 470.0 l Wasser versetzt. Nach dem Abkühlen des Reaktionsgemisches auf eine Temperatur von ca. 25°C werden 235.0 l Cyclohexan und 57.1 l Eisessig zu. Es wird nach ca. 1 h bei einer Temperatur im Intervall von 10 - 25°C nachgerührt. Die resultierende Suspension wird geschleudert und das Schleudergut anschließend mit 235.0 l Methyl-tert.-butylether daraufhin mit 353.5 l Wasser und abschließend mit 235 l Ethanol gewaschen. Man isoliert auf diese Weise nach dem Trocknen 90.6 kg (81.7%d. Th.) des 11-Cyclopropyl-5,11-dihydro-4-methyl-6H-dipyrido[3,2-b:2',3'-e][1,4]diazepin-6-ons (Nevirapine).

### Beispiel 3

287.2 kg 2-Chlor-N-(2-chlor-4-methyl-3-pyridyl)-3-pyridincarboxamid (4), 57.0 kg Calciumoxid und 87.1 kg Cyclopropylamin (Molverhältnis: 1:1:1,5) werden in 574 l Diglyme (Diethylenglykol-dimethylether) in einem 1200 1-VA-Rührwerksapparat für ca. 30 min auf 135-145°C erhitzt, wobei sich ein Druck von 1.2-1.5 bar aufbaut und ca. 50 % Ausgangsmaterials (4) abreagieren. Hierzu werden in ca. 30 min bei 135-145°C weitere 58.1 kg Cyclopropylamin zugepumpt, wobei sich ein Druck von 3.0-3.5 bar einstellt und weitere 25 % das Edukts (4) abreagieren. Die Mischung wird dann weiter für einen Zeitraum von 5 bis 6 h bei 135 bis 145°C gehalten. Anschließend wird das Reaktionsgemisch auf eine Temperatur von 20 bis 30°C abgekühlt und filtriert. Der Filterkuchen wird mit 144 l Diglyme gewaschen. Die Filtrate werden vereinigt und es werden zunächst 400 l Lösungsmittel abdestilliert. Der Rückstand wird daraufhin mit weiteren 287 l Diglyme verdünnt. Die so erhaltene verdünnte Lösung wird innerhalb eines Zeitraumes von 20 - 40 min. zu einer 130°C warmen Suspension von 110 kg 60-proz. Natriumhydrid in 862 l Diglyme zudosiert. Vorratsgefäß und Leitungen werden mit weiteren 144 l Diglyme gespült und das Gemisch wird unter Rühren über einen weiteren Zeitraum von 30 bis 60 min. auf einer Temperatur im Bereich von 130-140°C gehalten. Danach wird das Diglyme weitgehend abdestilliert; anschließend wird der verbliebene Rückstand vorsichtig mit 1150 l Wasser versetzt. Nach dem Abkühlen des Reaktionsgemisches auf eine Temperatur von ca. 25°C werden 575 l Cyclohexan und 147 l Eisessig zugefügt. Es wird nach ca. 1 h bei einer Temperatur in einem Intervall von 10 - 25°C nachgerührt. Die resultierende Suspension wird geschleudert und das Schleudergut anschließend mit 575 l Methyl-tert.-butylether und daraufhin mit 862 l Wasser und abschließend mit 575 l Ethanol gewaschen. Man isoliert auf diese Weise nach dem Trocknen 225 kg (83.0%d. Th.) des 11-Cyclopropyl-5,11-dihydro-4-methyl-6H-dipyrido[3,2-b:2',3'-e][1,4]diazepin-6-ons (Nevirapine).

## Patentansprüche

1. Verfahren zur Herstellung von Nevirapine, dadurch gekennzeichnet, daß man 2-Chlor-N-(2-chlor-4-methyl-3-pyridyl)-3-pyridincarboxamid mit Cyclopropylamin in Gegenwart von 0,5 bis 2 mol Calciumoxid in Diglyme über einen Zeitraum von 5 bis 8 Stunden auf eine Temperatur im Bereich von 130 bis 150°C erhitzt, die Reaktionsmischung anschließend auf eine Temperatur unterhalb 40°C abkühlt und filtriert, den Filterkuchen mit Diglyme wäscht, die vereinigten Filtrate einengt, den Rückstand mit Diglyme verdünnt und diese Lösung zu einer auf 120 bis 150°C erhitzten Lösung oder Suspension einer Base in Diglyme zudosiert und das Reaktionsgemisch über einen Zeitraum von 30 bis 90 Minuten auf einer Temperatur von 120 bis 150°C hält, danach das Reaktionsmedium weitestgehend abdestilliert und den Rückstand bei einer Temperatur im Bereich von 20 bis 90°C hydrolysiert, das Hydrolysat abkühlt und mit einem inerten Lösungsmittel und einer organischen Säure versetzt und die Mischung über einen Zeitraum von 30 bis 90 Minuten bei einer Temperatur in einem Intervall von 5 bis 50°C rührt, aus der resultierenden Suspension das Reaktionsprodukt abtrennt und zunächst mit einem inerten Lösungsmittel, nachfolgend mit Wasser und anschließend mit einem Alkanol wäscht und das Nevirapine isoliert.

2. Verfahren nach Anspruch 1, dadurch gekennzeichnet, daß man 2-Chlor-N-(2-chlor-4-methyl-3-pyridyl)-3-pyridincarboxamid mit Cyclopropylamin in Gegenwart von 1 bis 2 mol Calciumoxid in Diglyme über einen Zeitraum von 6 bis 7 Stunden auf eine Temperatur im Bereich von 135 bis 145°C erhitzt, die Reaktionsmischung anschließend auf eine Temperatur im Intervall von 20 bis 30°C abkühlt und filtriert, den Filterkuchen mit Diglyme wäscht, die vereinigten Filtrate einengt, den Rückstand mit Diglyme verdünnt und diese Lösung zu einer auf 130 bis 140°C erhitzten Lösung oder Suspension von Natriumhydrid in Diglyme zudosiert und das Reaktionsgemisch über einen Zeitraum von 30 Minuten bis zu einer Stunde auf einer Temperatur von 130 bis 140°C hält, danach das Reaktionsmedium weitestgehend abdestilliert und den Rückstand bei einer Temperatur im Bereich von 50 bis 80°C hydrolysiert, das Hydrolysat auf eine Temperatur von 25°C abkühlt und mit einem inerten Lösungsmittel und einer organischen Säure versetzt und die Mischung über einen Zeitraum von einer Stunde bei einer Temperatur in einem Intervall von 10 bis 25°C rührt, aus der resultierenden Suspension das Reaktionsprodukt abtrennt und zunächst mit einem inerten Lösungsmittel, nachfolgend mit Wasser und anschließend mit einem Alkanol wäscht und das Nevirapine isoliert.

3. Verfahren nach einem der Ansprüche 1 oder 2, dadurch gekennzeichnet, daß zunächst nur ein Teil des Cyclopropylamins eingesetzt wird, und der Rest erst im weiteren Verlauf der Amminierungsreaktion zugefügt wird.

4. Verfahren nach Anspruch 3, dadurch gekennzeichnet, daß von 250 mol-% Cyclopropylamin zunächst nur 100 bis 150 mol-% eingesetzt werden.

5. Verfahren nach einem der Ansprüche 1 bis 4, dadurch gekennzeichnet, daß man das Reaktionsmedium nach der Aminierungsreaktion abdestilliert und in einem weiteren Ansatz einsetzt.

## Claims

1. Process for preparing nevirapine, characterised in that 2-chloro-N-(2-chloro-4-methyl-3-pyridyl)-3-pyridine carboxamide is heated to a temperature in the range from 130 to 150°C with cyclopropylamine in the presence of 0.5 to 2 mol of calcium oxide in diglyme for a period of 5 to 8 hours, the reaction mixture is subsequently cooled to a temperature below 40°C and filtered, the filter cake is washed with diglyme, the combined filtrates are evaporated down, the residue is diluted with diglyme and this solution is metered into a solution or suspension of a base in diglyme which has been heated to 120 to 150°C and the reaction mixture is kept at a temperature of 120 to 150°C for a period of 30 to 90 minutes, then the reaction medium is largely distilled off and the residue is hydrolysed at a temperature in the range from 20 to 90°C, the hydrolysate is cooled and combined with an inert solvent and an organic acid and the mixture is stirred for a period of 30 to 90 minutes at a temperature in the range from 5 to 50°C, the reaction product is separated from the resulting suspension and washed first with an inert solvent, then with water and subsequently with an alkanol and the nevirapine is isolated.

2. Process according to claim 1, characterised in that 2-chloro-N-(2-chloro-4-methyl-3-pyridyl)-3-pyridine carboxamide is heated to a temperature in the range from 135 to 145°C with cyclopropylamine in the presence of 1 to 2 mol of calcium oxide in diglyme for a period of 6 to 7 hours, the reaction mixture is subsequently cooled to a temperature in the range from 20 to 30°C and filtered, the filter cake is washed with diglyme, the combined filtrates are evaporated down, the residue is diluted with diglyme and this solution is metered into a solution or suspension of sodium hydride in diglyme which has been heated to 130 to 140°C and the reaction mixture is kept at a temperature of 130 to 140°C for a period of from 30 minutes to one hour, then the reaction medium is largely distilled off and the residue is hydrolysed at a temperature in the range from 50 to 80°C, the hydrolysate is cooled to a temperature of 25°C and combined with an inert solvent and an organic acid and the mixture is stirred for a period of one hour at a temperature in the range from 10 to 25°C, the reaction product is separated from the resulting suspension and washed first with an inert solvent, then with water and subsequently with an alkanol and the nevirapine is isolated.

3. Process according to one of claims 1 or 2, characterised in that initially only some of the cyclopropylamine is put in and the remainder is only added later on in the amination reaction.

4. Process according to claim 3, characterised in that, out of 250 mol-% of cyclopropylamine, only 100 to 150 mol-% are put in initially.

5. Process according to one of claims 1 to 4, characterised in that the reaction medium is distilled off after the amination reaction and used in another batch.

## Revendications

1. Procédé de préparation de la névirapine, caractérisé en ce que l'on chauffe le 2-chloro-N-(2-chloro-4-méthyl-3-pyridyl)-3-pyridinecarboxamide avec la cyclopropylamine en présence de 0,5 à 2 moles d'oxyde de calcium dans le diglyme pendant une durée de 5 à 8 heures à une température située dans le domaine de 130 à 150°C, puis on refroidit le mélange réactionnel à une température inférieure à 40°C et on le filtre, on lave le gâteau de filtration avec du diglyme, on concentre les filtrats réunis, on dilue le résidu avec du diglyme et on ajoute cette solution à une solution ou suspension d'une base dans le diglyme chauffée à 120 à 150°C et on maintient le mélange réactionnel à une température de 120 à 150°C pendant une durée de 30 à 90 minutes, puis on élimine le milieu réactionnel le plus totalement possible par distillation et on hydrolyse le résidu à une température située dans le domaine de 20 à 90°C, on refroidit l'hydrolysat et on lui ajoute un solvant inerte et un acide organique et on agite le mélange pendant une durée de 30 à 90 minutes à une température située dans un intervalle de 5 à 50°C, on sépare le produit réactionnel de la suspension résultante et on le lave d'abord avec un solvant inerte, puis avec de l'eau et ensuite avec un alcanol et on isole la névirapine.

2. Procédé selon la revendication 1, caractérisé en ce que l'on chauffe le 2-chloro-N-(2-chloro-4-méthyl-3-pyridyl)-3-pyridinecarboxamide avec la cyclopropylamine en présence de 1 à 2 moles d'oxyde de calcium dans le diglyme pendant une durée de 6 à 7 heures à une température située dans le domaine de 135 à 145°C, puis on refroidit le mélange réactionnel à une température située dans l'intervalle de 20 à 30°C et on le filtre, on lave le gâteau de filtration avec du diglyme, on concentre les filtrats réunis, on dilue le résidu avec du diglyme et on ajoute cette solution à une solution ou suspension d'hydrure de sodium dans le diglyme chauffée à 130 à 140°C et on maintient le mélange réactionnel à une température de 130 à 140°C pendant une durée de 30 minutes à une heure, puis on élimine le milieu réactionnel le plus totalement possible par distillation et on hydrolyse le résidu à une température située dans le domaine de 50 à 80°C, on refroidit l'hydrolysat à une température de 25°C et on lui ajoute un solvant inerte et un acide organique et on agite le mélange pendant une durée d'une heure à une température située dans un intervalle de 10 à 25°C, on sépare le produit réactionnel de la suspension résultante et on le lave d'abord avec un solvant inerte, puis avec de l'eau et ensuite avec un alcanol et on isole la névirapine.

3. Procédé selon l'une des revendications 1 et 2 caractérisé en ce que l'on n'utilise d'abord qu'une partie de la cyclopropylamine et on n'ajoute le reste que dans la suite de la réaction d'amination.

4. Procédé selon la revendication 3 caractérisé en ce que, de 250 mol% de cyclopropylamine, on n'utilise d'abord que 100 à 150 mol%.

5. Procédé selon l'une des revendications 1 à 4 caractérisé en ce que l'on élimine le milieu réactionnel par distillation après la réaction d'amination et on l'utilise dans une opération ultérieure.
